# EUROPEAN PATENT APPLICATION

(11) **EP 0 543 738 A1**
(43) Date of publication of application: **26.05.1993**
(21) Application number: 92420145.2
(22) Date of filing: 05.05.1992
(51) Int. Cl.: A61B 1/00

(54) **Detachable servo actuated insertion tube for borescope or endoscope**

(30) Priority: 22.11.1991 US 796026
(71) Applicant: WELCH ALLYN, INC., Skaneateles Falls New York 13153 (US)
(72) Inventor: Pileski, Michael J., Skaneateles - NY 13152 (US)
(74) Representative: Laurent, Michel

(57) **Abstract**

An electrical servo control actuated insertion tube assembly (12) that is detachable from the processor control module (249 is provided. A joystick (38) actuated control handle (18) is connected to the proximal end of the insertion tube (14) and carries therein the servo control motors (26,28) for manipulation of the distal end through a pair of cables (32,34) attached thereto. The control handle (18) is connected electrically and optically through a flexible umbilical cord (20) to the control/processor unit (24) through an interface connector (22) to provide a flexible and easily actuated insertion tube (14) for borescope/endoscope use.

## Description

### Background of the Invention

This invention relates generally to borescopes or endoscopes having elongated insertion tubes for providing full color video images of inaccessible objects of the type having an articulated distal end of the insertion tube actuated by cable steering means and more particularly to an electrically actuated steerable insertion tube assembly.

A borescope is generally characterized as an elongated flexible insertion tube with a viewing head at its distal end and a control and processing section at its proximal end. The control section has, in addition to the power supply, light source, processing and video control included one or two pair of control cables extending to the articulated bendable tube section adjacent the viewing head for manipulation of the insertion tube as it is inserted into a remote inaccessible location. The steering mechanisms for these devices originally involved a rack and pinion cable actuation mechanism with control knobs for manipulation of the insertion tube. Generally the two pairs of cables have been disposed approximately ninety degrees to each other for steering in two planes. One or both pairs of these cables are differentially displaced for bending the steering section to facilitate the inspection of the object. Various devices have also been provided in the prior art for realizing a full color video picture of a target situated within a remote cavity. These devices have been gradually improved over time until today most devices of this type employ an external light source conveyed to the image viewing head by fiber optic bundles located within the insertion tube together with a solid state image sensor and lens system positioned in the distal end of the insertion tube which are connected to the external video processing system and a standard television format display.

An electrically actuated steering control system has been provided to make the steering of the distal end of the insertion tube more reliable and easier to accomplish. Such a system is shown in various patents owned by a common assignee of the present applicant, such as Wood et al. U.S. Pat No 4,941,454 and 4,941,456.

As part of the need for greater flexibility and portability, the control of the steering function of a borescope insertion tube has had to be simplified and improved both from operating and adjustability standpoints as well as sterilization and maintenance standpoints. Not only is it important for a single person to be able to easily operate a compact portable system from a variety of locations relative to the main processing module, but the requirement for precise positioning and for obtaining access through very tortuous channels in industrial products has created the need for an even more flexible and economical control system from that disclosed in the aforementioned patents.

### Objects and Summary of the Invention

It is therefore an object of the present invention to provide a steerable insertion tube for borescopes and endoscopes that overcomes the limitations of the prior art.

It is another object of the present invention to provide a detachable electrically actuated insertion tube for borescopes and endoscopes.

It is another object of the present invention to provide a servo motor controlled steerable borescope/endoscope insertion tube that requires only electrical and optical connection to a main processing control system of a borescope/endoscope.

It is another object of the present invention to provide a steerable insertion tube and control for a borescope/endoscope that can be readily rotated physically in addition to be being articulated at the distal end.

It is another object of the present invention to provide a detachable insertion tube having an articulated steering control for the distal end, that can be detached from a control and processing system for sterilization and the like.

It is another object of the present invention to provide a simplified and flexible steering control system for a borescope/endoscope insertion tube in which the distal end is articulated for insertion and observation purposes.

It is a still further object of the present invention to provide a steerable insertion tube for borescopes/endoscopes in which the articulated distal end can be manipulated to a bent condition and held in the bent condition.

It is another object of the present invention to provide an insertion tube having an electrically actuated steerable distal end with a control and actuation system for cable actuation thereof in which the cables are protected from application of excessive force either by the control and actuation system or by accidental manual manipulation of the distal end.

In one embodiment, the foregoing objects and objectives are achieved by an elongated flexible cable actuated insertion tube having a handle and joystick control fixed at the proximal end of the insertion tube and connected to a processor control and display system by a flexible electrical/optical umbilical cord only, which is disconnectable at the processor display module.

### Brief Description of the Drawings

For a better understanding of these and other objects of the present invention, reference is made to the detailed description of the invention which is to be read in conjunction with the following drawings, wherein:
Fig. 1 is a perspective view of a system according to the present invention;
Fig. 2 is a perspective view of the control handle for the insertion tube of the present invention; and
Fig. 3 is a block diagram of the operational control of a borescope/endoscope insertion tube according to the present invention.

### Detailed Description of a Preferred Embodiment

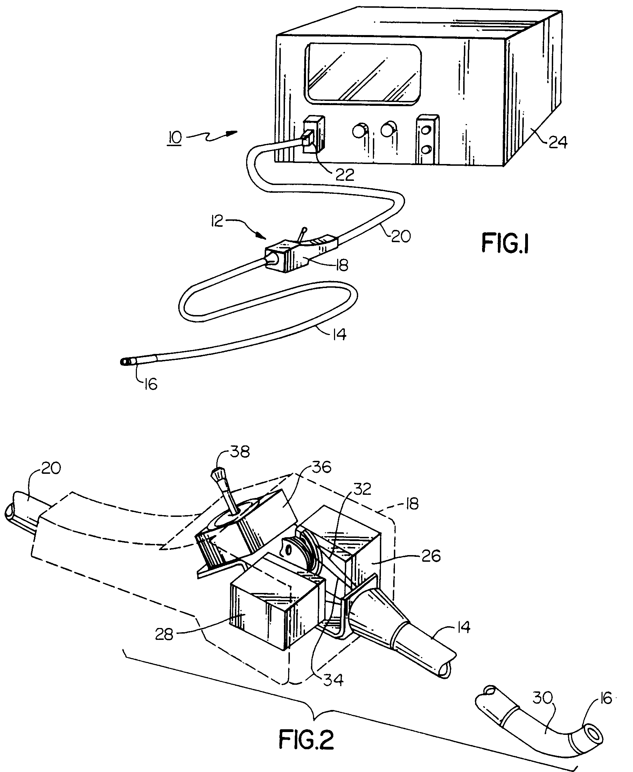

Referring now to Fig. 1, a borescope/endoscope 10 according to the present invention includes a detachable insertion tube assembly 12 which has a flexible insertion tube portion 14 with the usual viewing head 16 at the distal end thereof and which is connected to an actuating handle assembly 18 joined by umbilical cord 20 to a disconnect/interface connector 22 plugged into the processor display unit 24. The complete insertion tube assembly 12 can be detached from the processor unit 24 for packaging or other purposes such as sterilization of the insertion tube portion 14 in endoscope applications. The assembly 12 is completely self-contained, connected electrically and optically to the processor unit 24 but requiring no mechanical attachment.

As may be seen in Fig. 2, the insertion tube 14 is connected to the handle 18 at its proximal end. The handle 18 has mounted therein a pair of servo control motors 26 and 28 each connected to a pair of actuating cables 32 and 34 which extend through the insertion tube 14 to the articulated section 30 of the insertion tube 14 which carries thereon the viewing head 16. The pairs of cables 32 and 34 are generally disposed at right angles to each other at section 30 so as to provide full 360 degree manipulation of the articulation section 30 of the insertion tube 14.

Also mounted in the handle assembly 18 is a joystick module 36 which includes the joystick 38 and the circuitry for applying proper voltages to the motors 26 and 28 as the joystick 38 is manipulated. Electrical power for the joystick assembly 36 is provided through the umbilical cord 20 to apply the proper voltages, corresponding to the position of the joystick 38, to the respective servo motors 26 and 28 for the desired actuation of the articulations section of the distal end of the insertion tube 14.

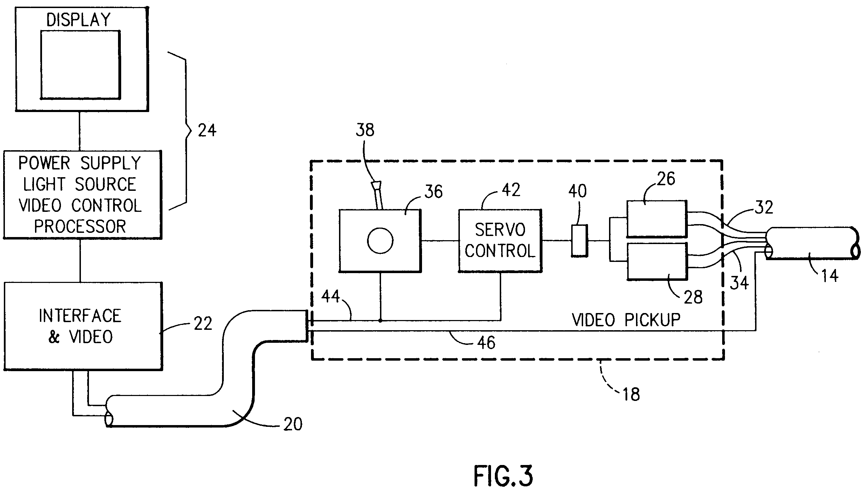

Referring now to Fig. 3 there is shown in block diagram form the control circuits for this detachable insertion tube assembly 12. As may be seen, the handle assembly 18 is shown in dotted line as a box to which is connected the insertion tube 14 with the two pairs of cables 32 and 34 being connected to the servo motors 26 and 28. For a detailed description of how the cables are connected to a servo motor, reference is made to the aforementioned patents which show in complete detail a suitable connection and drive arrangement. The two servo motors 26 and 28 are electrically connected through a "park" module 40 to the main servo control circuit 42. The servo control 42 is connected to the joystick assembly 36 for application of the position control voltages to the servo motors 26 and 28 in accordance with the position of the joystick handle 38. Power for the joystick assembly 36 and the servo control 42 is provided through the electrical conductor 44 which extends through the umbilical cord 20, and the interface connector 22 to the power supply in the processor unit 24. The light input to the viewing head 16 as well as the video pickup return are shown collectively by the line 46 which extends from the viewing head 16 back through the handle 18, the umbilical cord 20, and the interface connector 22 to the processor/display 24.

In use, the insertion tube assembly 12 can be simply and easily handled by an individual operator. The insertion tube 14 and viewing head 16 can be guided into the desired location within the object to be inspected by actuation of the joystick 38. In addition, the entire handle assembly 18 can be rotated to assist in the guiding of the viewing head 16 at the distal end of the insertion tube 14 as it is guided into the remote inaccessible location to be viewed. Light is provided via optical fibers from the processor control unit 24 through the umbilical and the insertion tube 14 in the usual manner. The video pickup is returned similarly via optical fiber cables to the processor unit. The entire insertion tube assembly 12 can be disconnected at the interface connector 22 for packaging and/or for sterilization of the insertion tube 14 when required. Also, with this configuration, if a different type of insertion tube is required, either as to size, lens capability or whatever, a separate unit can be plugged in at the interface connector 22 without having to completely change the entire borescope/endoscope system. This greatly adds to the flexibility of the overall system.

Actuation of the joystick 38 applies power to the two servo motors 26 and 28, through the servo control 42. Control 42 includes a power limiting circuit setable to ensure that the elastic limit of the cables 32 and 34 are not exceeded in the actuation of the articulated end of the insertion tube. This limiting circuit ensures that excess force is not applied when the viewing head 16 encounters physical obstacles in the process of being inserted or withdrawn from the desired location. In this way, the frequent abuse of the cable actuation system is prevented and the constant need to readjust and refurbish the steering control section of the insertion tube is eliminated.

In addition, it has been found desirable to provide the ability to electrically "park" the actuated tip 16 in a desired attitude for viewing so that the joystick 38 does not have to be held in the actuated position to ensure that the viewing head 16 will remain oriented as desired. With the system shown in the prior referenced applications, control pressure on the joystick 38 has to be maintained to maintain the viewing head 16 at the distal end of the articulation section 30 in a bent condition. With the provision of the "park" control circuit 40, after the viewing head 16 is positioned as desired by manipulation of the joystick lever 38, a control button is actuated on the circuit 40 and this will then hold the tube in the actuated condition and the joystick 38 can be released. This function is accomplished in a preferred form of the present invention by the removal of power to the servo motors, thus allowing them to stop in an actuated condition with the normal internal frictional resistance of the cables in the elongated insertion tube holding the viewing head 16 in the bent condition. Alternatively, of course, a holding voltage could be applied to the appropriate servo motors 26 and 28 to maintain the articulation without continued application of manual pressure to the joystick 38. The foregoing "power off" circuit has an additional advantage in that once the viewing head 16 is bent into the desired location, accidental withdrawal without returning it to neutral will not rupture an actuating cable. The internal friction of the bent tube can usually be overcome without damage to the cable pairs 32 or 34 by application of straightening pressure to the bent articulated section 30. The electric "park" circuit 40 also includes, in the preferred embodiment, a circuit for automatically returning the articulation section 30 to a straight neutral position upon reenergizing of the servo control system after parking operation. Suitable control switches and lights [not shown] on the handle assembly 18 alert the operator of the device as to the condition of the tip of the insertion tube.

It will thus be seen that I have provided a very flexible and economical to manufacture detachable insertion tube assembly for use in borescopes and endoscopes which greatly enhances the utility and flexibility of use of such systems. The convenience of electrical servo actuation of the insertion tube is retained without the necessity of being attached to the processor control unit and the flexibility and convenience of the former all manual systems is also retained.

While this invention has been explained with reference to the structure disclosed herein, it is not confined to the details set forth and this application is intended to cover any modifications and changes as may come within the scope of the following claims:

## Claims

1. A portable servo motor actuated cable controlled insertion tube borescope/endoscope of the type having an insertion tube with a cable bendable steering section and viewing head at the distal end, steering cables connected from the distal steering section to servo motors at the proximal end of the insertion tube, and illumination and image viewing channels extending from the distal end to the proximal end wherein the improvement comprises:
a handle member (18) having at least one servo motor (26) mounted in said handle member (18);
a steerable insertion tube (14) having a viewing head (16) at the distal end and connected at the proximal end to said handle member (18);
at least one pair of actuating cables (32) connected at one end to the distal end of said insertion tube and at the other end to said servo motor (26);
control means (38) mounted in said handle member (18) operatively connected to said at least one servo motor (26) for energizing said servo motor to cause said actuating cables to bend the distal end of said insertion tube (14);
light, power and image cable means (46) extending from said handle member (18) and detachably connected to processor, light source and power supply means (24) remote from said handle member (18);
whereby a servo motor actuated insertion tube can be quickly and easily attached to and detached from a borescope/endoscope apparatus for storage, sanitizing or other purposes.

2. A borescope/endoscope according to claim 1 further characterized by two electric servo motors (26,28) mounted in said handle member (18) and two pairs of cables (32,34) disposed substantially at right angles to each other and connected between the distal end of said insertion tube and said servo motors for 360° electric manipulation of the viewing head.

3. A borescope/endoscope according to claim 1 wherein the light, power and image cable means comprise a flexible and twistable umbilical cord (20) so that the control handle (18) and insertion tube (14) can be freely turned about the axis thereof to help guide said insertion tube into the object to be viewed at an inspection site.

4. A borescope/endoscope according to claim 2 further characterized by circuit means for selectively electrically parking said servo motors after being actuated by said control means to hold the distal end of said insertion tube in a bent condition.

5. A borescope/endoscope according to claim 2 wherein said control means (38) includes steering circuit means (36) positioned in said control handle to actuate said servo motors (26,28) in accordance with the position of said actuating means (38).

6. A borescope/endoscope according to claim 5 wherein said steering circuit means includes power limiting circuits connected in said servo motor actuating means to prevent excessive force being applied by said servo motors to the actuating cables.

7. A borescope/endoscope according to claim 5 wherein said steering circuit means (36) includes a power interrupt circuit for selectively removing actuating power from said servo motors (26,28) so that manual movement of the distal end of the insertion tube will not damage the operating cables.

8. A borescope/endoscope according to claim 7 wherein said power interrupt circuit includes means to automatically center the servo motors upon reapplication of servo power.

9. A borescope/endoscope according to any of claims 1-8 further characterized by a joystick control lever (38) and circuit (36) for applying an operating voltage to said servo motors (26,28) proportional to the displaced position of said joystick (38) in said control.
